# EUROPEAN PATENT APPLICATION

(11) **EP 4 613 878 A1**
(43) Date of publication of application: **10.09.2025**
(21) Application number: 24382240.0
(22) Date of filing: 05.03.2024
(51) Int. Cl.: C12Q 1/6883, C12Q 1/689, C12Q 1/70

(54) **IN VITRO METHOD FOR THE DIFFERENTIAL DIAGNOSIS BETWEEN VIRAL AND BACTERIAL PNEUMONIA IN A PATIENT**

(71) Applicant: Fundación Pública Galega Instituto de Investigación Sanitaria de Santiago de Compostela, 15706 Santiago de Compostela A Coruña (ES); Servizo Galego De Saúde, 15703 Santiago de Compostela, A Coruna (ES); Universidade de Santiago de Compostela, 15782 Santiago de Compostela (ES)
(72) Inventor: GÓMEZ CARBALLA, Alberto, 15706 Santiago de Compostela (ES); VIZ LASHERAS, Sandra, 15706 Santiago de Compostela (ES); MARTINÓN TORRES, Federico, 15706 Santiago de Compostela (ES); SALAS ELLACURIAGA, Antonio, 15706 Santiago de Compostela (ES)
(74) Representative: Hoffmann Eitle

(57) **Abstract**

The present invention refers to an *in vitro* method for identifying biomarkers or biomarker signatures for the differential diagnosis between viral and bacterial pneumonia in a patient, for the differential diagnosis between viral and bacterial pneumonia in a patient and/or for selecting a therapy for a patient suffering from pneumonia.

## Description

### FIELD OF THE INVENTION

The present invention refers to the medical field. Particularly, it refers to an *in vitro* method for identifying biomarkers or biomarker signatures for the differential diagnosis between viral and bacterial pneumonia in a patient, for the differential diagnosis between viral and bacterial pneumonia in a patient and/or for selecting a therapy for a patient suffering from pneumonia.

### STATE OF THE ART

Pneumonia is an acute respiratory infection, and one of the leading causes of morbidity and mortality worldwide. In children younger than 5 (excluding the neonatal period), it remains the primary cause of death. Common symptoms in children include fever, tachypnoea, cough, chest pain and difficulty in breathing. Pneumonia can be caused by a variety of microorganisms, including bacteria, viruses and fungi, depending on age groups and the specific epidemiological situation. However, *Streptococcus pneumoniae* is the most common bacterial cause of pneumonia across all age group. The causative microorganisms also vary significantly between the two main types of pneumonia: Community-Acquired Pneumonia (CAP) and Hospital-Acquired Pneumonia (HAP). *Pseudomonas aeruginosa, Staphylococcus aureus,* and *Enterobacter* are the most common causes of HAP whereas *Streptococcus pneumoniae, Staphylococcus aureus, Streptococcus pyogenes, Haemophilus influenzae*, *Mycoplasma pneumoniae, Legionella pneumophila* and respiratory viruses, such as Respiratory Syncytial virus, Rhinovirus or Influenza virus A/B, are the most common microorganisms responsible for CAP. The introduction of improved conjugate vaccines against Haemophilus influenzae type b and *Streptococcus pneumoniae* has led to a reduction in the incidence and severity of childhood pneumonia, resulting in significant changes in the proportions of CAP cases etiologies.

The clinical presentation of pneumonia is diverse and may overlap with other respiratory conditions such as asthma, bronchiolitis, pertussis, lung abscess, bronchiectasis, or malaria, among others. Accurate assessment of disease severity is crucial for effective management, influencing decisions on antibiotic prescriptions and hospitalization. Despite efforts to enhance international guidelines, the variability in causative microorganisms and symptoms poses a significant challenge for clinicians in healthcare centres. Current diagnostic criteria rely on nonspecific symptoms, chest imaging, and inconclusive laboratory analysis, with limitations in differentiating the etiology of the disease (i.e., viral, bacterial). Obtaining lower respiratory tract samples in children is often difficult. Consequently, misdiagnosis of bacterial pneumonia is common, leading to increased costs, unnecessary medical tests, hospital admissions, and incorrect antibiotic prescriptions.

There is an unmet medical need of finding reliable biomarkers aimed at differentially diagnosing between viral and bacterial pneumonia in a patient. This would provide clinicians with a new tool for precisely selecting a therapy depending on whether the patient is suffering from viral or bacterial pneumonia. The present invention is focused on solving this problem and an innovative method for the differential diagnosis between viral and bacterial pneumonia in a patient is herein provided.

### DESCRIPTION OF THE INVENTION

### Brief description of the invention

The present invention refers to an *in vitro* method for identifying biomarkers or biomarker signatures for the differential diagnosis between viral and bacterial pneumonia in a patient, for the differential diagnosis between viral and bacterial pneumonia in a patient and/or for selecting a therapy for a patient suffering from pneumonia.

Host transcriptomic biomarkers were identified in the blood of patients, particularly children, affected by viral and bacterial pneumonia, alongside healthy controls. The main goal was to establish a gene-expression signature enhancing disease diagnosis and management. The inventors of the present invention conducted an analysis of a total of 192 whole blood samples, comprising 38 controls and 154 viral and bacterial pneumonia patients recruited through the EUCLIDS clinical network. 5,486 differentially expressed genes (DEGs) were identified when comparing blood RNA from pneumonia patients with healthy controls. Functional enrichment analysis highlighted pathways related to the immune system response, encompassing neutrophil degranulation, humoral immune response, and various inflammatory pathways. In the comparative analysis of gene expression between viral and bacterial pneumonia patients, 272 DEGs were identified. Gene set analysis revealed a significant difference in pathway enrichment for the immune response, contingent on the over-regulation of gene sets in viral or bacterial pneumonias.

Additionally, we identified a 5-transcript host signature specifically designed to distinguish between viral and bacterial pneumonia. Such as it is shown in **Table 1,** genes *MXRA7*, *FAM20A, BAG3, TDRD9* or *KLF14* can be individually used to discriminate between viral and bacterial pneumonia. Moreover, combinations of these genes comprising two, three, four or five genes gave rise to high ranked signatures showing a better performance to discriminate between viral and bacterial pneumonia.

**Table 1**

| | | |
|---|---|---|
| **Genes** | **AUC [CI]** | **Sensitivity/Specificity** |
| ***MXRA7*** | 0.89 [0.81- 0.98] | 0.82/1.00 |
| ***TDRD9*** | 0.85 [0.68-1.00] | 0.82/0.89 |
| ***BAG3*** | 0.73 [0.50-0.96] | 0.87/0.67 |
| ***KLF14*** | 0.88 [0.79-0.97] | 0.77/1.00 |
| **FAM20A** | 0.86 [0.74-0.98] | 0.75/0.89 |
| ***MXRA7, TDRD9*** | 0.93 [0.83-1.00] | 0.85/0.89 |
| ***TDRD9,BAG3*** | 0.92 [ 0.85-0.99] | 0.80/1.00 |
| ***MXRA7, BAG3*** | 0.91 [ 0.83-0.99] | 0.75/1.00 |
| ***MXRA7, FAM20A*** | 0.90 [0.81-0.98] | 0.82/1.00 |
| ***MXRA7, KLF14*** | 0.82 [0.69-0.94] | 0.65/1.00 |
| ***TDRD9, KLF14*** | 0.82 [0.63-1.00] | 0.82/0.89 |
| ***TDRD9, FAM20A*** | 0.85 [0.67-1.00] | 0.85/0.89 |
| ***BAG3, KLF14*** | 0.88 [ 0.76-1.00] | 0.85/0.89 |
| ***BAG3, FAM20A*** | 0.87 [0.76-0.98] | 0.77/0.89 |
| ***KLF14, FAM20A*** | 0.87 [0.77-0.97] | 0.77/1.00 |
| ***TDRD9, FAM20A, KLF*** | 0.83 [0.63-1.00] | 0.82/0.89 |
| ***TDRD9, BAG3, FAM20A*** | 0.92 [0.84-1.00] | 0.80/1.00 |
| ***TDRD9, BAG3, KLF14*** | 0.90 [0.79-1.00] | 0.87/0.89 |
| ***MXRA7, BAG3, KLF14*** | 0.88 [0.78-0.99] | 0.67/1.00 |
| ***MXRA7, BAG3, FAM20A*** | 0.91 [0.83-1.00] | 0.75/1.00 |
| ***MXRA7, TDRD9, FAM20A*** | 0.93 [0.83-1.00] | 0.85/0.89 |
| ***MXRA7, TDRD9, KLF14*** | 0.92 [0.82-1.00] | 0.95/0.78 |
| ***MXRA7, TDRD9, BAG3*** | 0.95 [0.88-1.00] | 0.80/1.00 |
| ***MXRA7, TDRD9, BAG3, KLF14*** | 0.95 [0.88-1.00] | 0.80/1.00 |
| ***MXRA7, TDRD9, BAG3, FAM20A*** | 0.95 [0.88-1.00] | 0.80/1.00 |
| ***MXRA7,BAG3, KLF14, FAM20A*** | 0.89 [0.78-0.99] | 0.67/1.00 |
| ***TDRD9, BAG3, FAM20A, KLF14*** | 0.89 [0.79-1.00] | 0.87/0.89 |
| ***MXRA7, TDRD9, FAM20A, KLF14*** | 0.92 [0.81-1.00] | 0.82/0.89 |
| ***MXRA7, TDRD9, FAM20A, KLF14, BAG3*** | 0.95 [0.88-1.00] | 0.80/1.00 |

Particularly, such as it is shown in the results (see **Example 2**) a 5-transcript host signature was identified which is specifically designed to distinguish between viral and bacterial pneumonia (particularly in pediatric pneumonia): *FAM20A, BAG3, TDRD9, MXRA7* and *KLF14*; AUC: 0.95 [0.88-1.00]), pseudo-validated in a cohort including probable bacterial and viral patients (AUC: 0.87 [0.77-0.97]). This signature holds the potential to enhance the accuracy of previously described general transcript-based signatures for viral and bacterial infections.

So, the first embodiment of the present invention refers to an *in vitro* method for identifying biomarkers or biomarker signatures for the differential diagnosis between viral and bacterial pneumonia in a patient, which comprises assessing the level of expression of at least a gene selected from the group consisting of: *MXRA7*, *FAM20A, BAG3, TDRD9* or *KLF14,* in a biological sample obtained from the patient, wherein the identification of a deviation of the level of expression of the gene/s as compared with a pre-established level of expression measured in a biological sample obtained from patients with viral pneumonia, is an indication that the gene/s may be used as biomarker or biomarker signatures for the differential diagnosis between viral and bacterial pneumonia.

The second embodiment of the present invention refers to an *in vitro* method for the differential diagnosis between viral and bacterial pneumonia in a patient, which comprises assessing the level of expression of at least a gene selected from the group consisting of: *MXRA7, FAM20A, BAG3, TDRD9* or *KLF14* in a biological sample obtained from the patient, wherein the identification of an over-expression of the genes *MXRA7*, *FAM20A, TDRD9* or *KLF14*, or an under-expression of the gene *BAG3,* as compared with a pre-established level of expression measured in a biological sample obtained from patients with viral pneumonia, is an indication that the patient is suffering from bacterial pneumonia; or wherein the identification of an under-expression of the genes *MXRA7*, *FAM20A, TDRD9* or *KLF14*, or an over-expression of the gene *BAG3,* as compared with a pre-established level of expression measured in a biological sample obtained from patients with bacterial pneumonia, is an indication that the patient is suffering from viral pneumonia.

The third embodiment of the present invention refers to an *in vitro* method for selecting a therapy for a patient suffering from pneumonia which comprises assessing the level of expression of at least a gene selected from the group consisting of: *MXRA7, FAM20A, BAG3, TDRD9* or *KLF14* in a biological sample obtained from the patient, wherein the identification of an over-expression of the genes *MXRA7, FAM20A, TDRD9* or *KLF14*, or an under-expression the gene *BAG3,* as compared with a pre-established level of expression measured in a biological sample obtained from patients with viral pneumonia, is an indication that the patient is suffering from bacterial pneumonia and consequently a treatment with antibiotics can be recommended; or wherein the identification of an under-expression of the genes *MXRA7*, *FAM20A, TDRD9* or *KLF14*, or an over-expression the gene *BAG3,* as compared with a pre-established level of expression measured in a biological sample obtained from patients with bacterial pneumonia, is an indication that the patient is suffering from viral pneumonia and consequently a treatment with antibiotics can be discarded.

The fourth embodiment of the present invention refers to the *in vitro* use of at least a gene selected from the group consisting of: *MXRA7, FAM20A, BAG3, TDRD9* or *KLF14*, or of a kit comprising reagent for assessing the level of expression of at least a gene selected from the group consisting of: *MXRA7, FAM20A, BAG3, TDRD9* or *KLF14*, for identifying biomarkers or biomarker signatures for the differential diagnosis between viral and bacterial pneumonia in a patient, for the differential diagnosis between viral and bacterial pneumonia in a patient or for selecting a therapy for a patient suffering from pneumonia.

The fifth embodiment of the present invention refers antibiotics for use in a method for the treatment of pneumonia wherein the method comprises determining whether the patient is suffering from bacterial pneumonia by assessing the level of expression of at least a gene selected from the group consisting of: *MXRA7*, *FAM20A, BAG3, TDRD9* or *KLF14* in a biological sample obtained from the patient, wherein the identification of an over-expression of the genes *MXRA7, FAM20A, TDRD9* or *KLF14*, or an under-expression the gene *BAG3,* as compared with a pre-established level of expression measured in a biological sample obtained from patients with viral pneumonia, is an indication that the patient is suffering from bacterial pneumonia and consequently a treatment with antibiotics can be recommended. Alternatively, the present invention refers to method for treating pneumonia with antibiotics which comprises administering a therapeutically effective dose or amount of the antibiotic to the patient, and wherein the method comprises, as first step, determining whether the patient is suffering from bacterial pneumonia by assessing the level of expression of at least a gene selected from the group consisting of: *MXRA7*, *FAM20A, BAG3, TDRD9* or *KLF14* in a biological sample obtained from the patient, wherein the identification of an over-expression of the genes *MXRA7*, *FAM20A, TDRD9* or *KLF14,* or an under-expression the gene *BAG3,* as compared with a pre-established level of expression measured in a biological sample obtained from patients with viral pneumonia, is an indication that the patient is suffering from bacterial pneumonia and consequently a treatment with antibiotics can be recommended.

In a preferred embodiment, the biomarker or biomarker signature is selected from **Table 1,** wherein the identification of an over-expression of the genes *MXRA7, FAM20A, TDRD9* or *KLF14,* or an under-expression the gene *BAG3,* as compared with a pre-established level of expression measured in a biological sample obtained from patients with viral pneumonia, is an indication that the patient if suffering from bacterial pneumonia; or wherein the identification of an under-expression of the genes *MXRA7, FAM20A, TDRD9* or *KLF14,* or an over-expression the gene *BAG3,* as compared with a pre-established level of expression measured in a biological sample obtained from patients with bacterial pneumonia, is an indication that the patient if suffering from viral pneumonia.

In a preferred embodiment, the signature comprises the combination of the genes: *MXRA7*, *FAM20A, BAG3, TDRD9* and *KLF14,* wherein the identification of an over-expression of the genes *MXRA7, FAM20A, TDRD9* and *KLF14,* and an under-expression the gene *BAG3,* as compared with a pre-established level of expression measured in a biological sample obtained from patients with viral pneumonia, is an indication that the patient if suffering from bacterial pneumonia; or wherein the identification of an under-expression of the genes *MXRA7*, *FAM20A, TDRD9* and *KLF14,* and an over-expression the gene *BAG3,* as compared with a pre-established level of expression measured in a biological sample obtained from patients with bacterial pneumonia, is an indication that the patient if suffering from viral pneumonia.

In a preferred embodiment, the biological sample is selected from blood, serum or plasma.

In a preferred embodiment, the antibiotic is selected from the group: ampicillin, ceftobiprole, ceflaroline, clindamycin, dalbavancin, daptomycin, linezolid, oritavancin, tedizolid, telavancin, tigecycline, vancomycin, aminoglycosides, carbapenems, ceftazidime, ceftobiprole, fluoroquinolines, piperacillin/tazobactam, ticarcillin/clavulanic acid, streptogramins, such as amikacin, gentamicin, kanamycin, netilmicin, tobramycin, paromomycin, streptomycin, geldanamycin, herbimycin, rifaximin, loracarbef, ertapenem, doripenem, imipenem/cilastatin, meropenem, cefadroxil, cefazolin, cefalotin/cefalothin, cefalexin, cefaclor, k cefamandole, cefoxitin, cefprozil, cefuroxime, cefixime, cefdinir, cefditoren, cefoperazone, cefotaxime, cefpodoxime, ceftazidime, ceftibuten, ceftizoxime, ceftriaxone, cefepime, ceftaroline fosamil, ceftobiprole, teicoplanin, vancomycin, telavancin, dalbavancin, oritavancin, dalbavancin, oritavancin, clindamycin, linomycin, daptomycin, azithromycin, clarithromycin, dirithromycin, erythromycin, roxithromycin, troleandomycin, telithromycin, spiramycin, aztreonam, furazilidone, linezolid, posizolid, radezolid, torezolid, amoxicillin, ampicillin, azlocillin, carbenicillin, cloxacillin, dicloxacillin, flucloxacillin, mezlocillin, nafcillin, oxacillin, penicillin G, penicillin V, piperacillin, temocillin, ticarcillin, amoxicillin/clavulanate, ampicillin/sulbactam, piperacillin/tazobactam, bacitracin, colistin, polymyxin B, ciprofloxacin, enoxacin, gatifloxacin, gemifloxacin, levofloxacin, lomefloxacin, moxifloxacin, nalidixic acid, norfloxacin, ofloxacin, trovafloxacin, grepafloxacin, sparfloxacin, temafloxacin, mafenide, sulfacetamide, sulfadiazine, silver sulfadiazine, sulfadimethoxine, sulfasalazine, sulfisoxazole, trimethoprim-sulfamethoxazole, sulfonamidochrysoidine, demeclocycline, doxycycline, minocycline, oxytetracycline, tetracycline, clofazimine, dapsone, capremycin, cycloserine, ethambutol, ethionamide, isoniazid, pyrazinamide, rifampicin, rifapentine, streptomycin, chloramphenicol, fosfomycin, fusidic acid, metronidazole, mupirocin, platensimycin, quinupristin/dalfopristin, thiamphenicol, tigecycline, tinidazole or trimethoprim.

In a preferred embodiment, bacterial infection is selected from: *Chlamydia pneumoniae, Chlamydia trachomatis, Chlamydophila psittaci, Mycoplasma pneumonia, Corynebacterium diphtheriae, Clostridium botulinum, Clostridium difficile, Clostridium perfringens, Clostridium tetani, Enterococcus faecalis, Enterococcus faecium, Listeria monocytogenes, Staphylococcus aureus, Staphylococcus epidermidis, Staphylococcus saprophyticus, Group B streptococcus, Streptococcus agalactiae, Streptococcus pneumoniae, Streptococcus pyogenes, or acid fast bacteria such as Mycobacterium leprae, Mycobaterium tuberculosis, Mycobacterium ulcerans, Mycobacterium avium intercellularae, Bordetella pertussis, Borrelia burgdorferi, Brucella abortus, Brucella canis, Brucella melitensis, Brucella suis, Campylobacter jejuni, Escherichia coli, Francisella tularensis, Haemophilus influenzae, Helicobacter pylori, Legionella pneumophila, Leptospira interrogans, Neisseria gonorrhoeae, Neisseria meningitidis, Pseudomonas aeruginosa, Pseudomonas spp, Rickettsia rickettsii, Salmonella typhi, Salmonella typhimurium, Shigella sonnei, Treponema pallidum, Vibrio cholerae, Yersinia pestis, Kingella kingae, Stenotrophomonas* and *Klebsiella.*

In a preferred embodiment, viral infection is selected from: Influenza such as Influenza A, including but not limited to: H1N1, H2N2, H3N2, H5N1, H7N7, H1N2, H9N2, H7N2, H7N3, H10N7, Influenza B and Influenza C, Respiratory Syncytial Virus (RSV), rhinovirus, enterovirus, bocavirus, parainfluenza, adenovirus, metapneumovirus, herpes simplex virus, Chickenpox virus, Human papillomavirus, Hepatitis, Epstein-Barr virus, Varicella-zoster virus, Human cytomegalovirus, Human herpesvirus, type 8 BK virus, JC virus, Smallpox, Parvovirus B 19, Human astrovirus, Norwalk virus, coxsackievirus, poliovirus, Severe acute respiratory syndrome virus, yellow fever virus, dengue virus, West Nile virus, Rubella virus, Human immunodeficiency virus, Guanarito virus, Junin virus, Lassa virus, Machupo virus, Sabia virus, Crimean-Congo haemorrhagic fever virus, Ebola virus, Marburg virus, Measles virus, Mumps virus, Rabies virus, Coronavirus and Rotavirus.

In a preferred embodiment, the present invention is a computer-implemented invention, wherein a processing unit (hardware) and a software are configured to: a) Receive the level of expression of the genes/signatures of **Table 1,** b) process the level of expression for finding substantial variations or deviations, and c) provide an output through a terminal display of the variation or deviation of the level of expression, wherein the variation or deviation of the level of expression may be used for the differential diagnosis between viral and bacterial pneumonia in a patient.

For the purpose of the present invention, the following terms are defined:
- The expression "deviation of the level of expression" refers to a statistically significant higher/lower level of expression, when comparing expression levels measured in patients suffering from bacterial or viral pneumonia.
- According to the present invention, a reference value can be a "pre-established threshold value" or a "cut-off' value. Typically, a "threshold value" or "cut-off value" can be determined experimentally, empirically, or theoretically. According to the present invention, the "pre-established threshold" value refers to a value previously determined in patients with bacterial or viral pneumonia. A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative). Typically, the optimal sensitivity and specificity (and so the "threshold value") can be determined using a Receiver Operating Characteristic (ROC) curve based on experimental data.
- The term "comprising" means "including", but not limited to what follows the term "comprising". Thus, the use of the term "comprising" indicates that the elements listed are necessary or mandatory, but that other elements are optional and may or may not be present.
- The term "consisting of" means "including" but is limited to what follows the term "consisting of". Thus, the term "consists of" indicates that the elements listed are mandatory, and that other elements may not be present.
- By "therapeutically effective dose or amount" of an antibiotic is intended an amount that, when administered as described herein, brings about a positive therapeutic response in a subject having bacterial pneumonia. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.

### Description of the figures

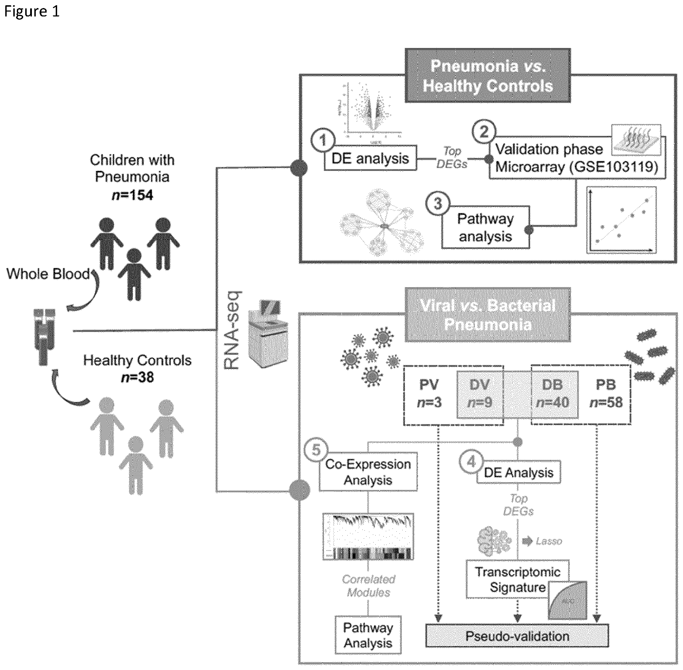

**Figure 1**. Scheme of the study design. The figure was built using Biorender resources (https://biorender.com/).

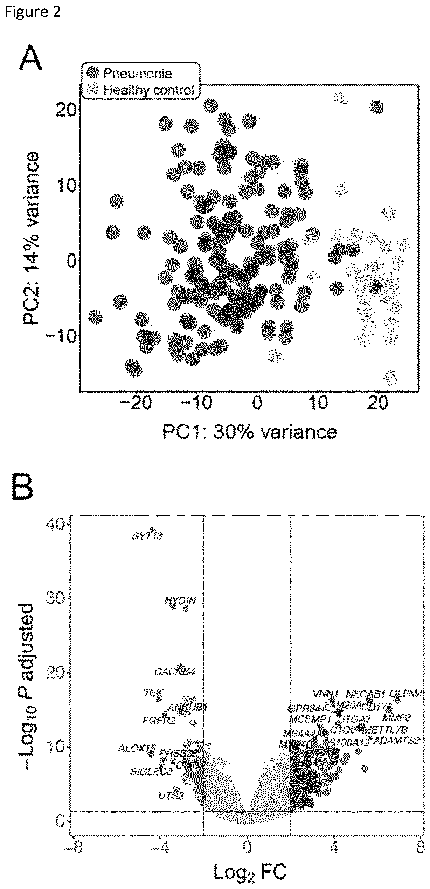

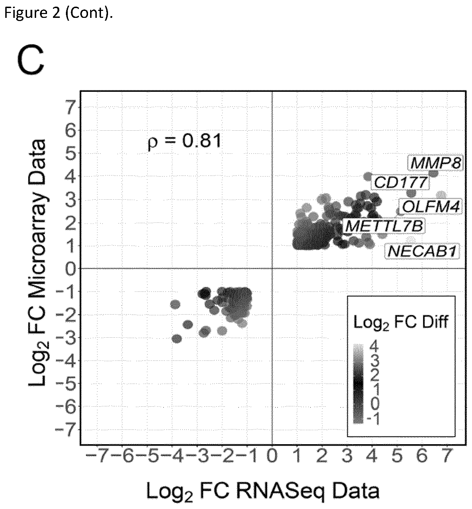

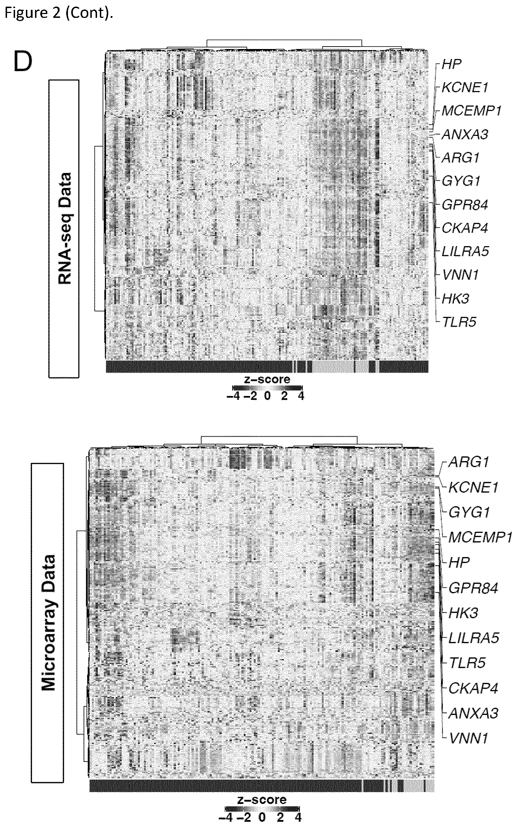

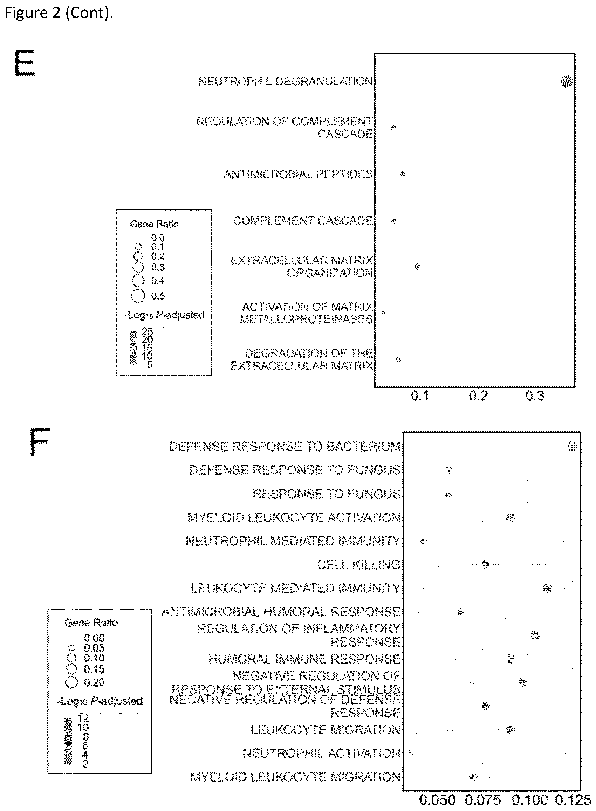

**Figure 2**. **(A)** PCA of transcriptome profiles from pneumonia and healthy control samples. Two first principal components (PC1 and PC2) are shown. **(B)** Volcano plot showing the DEGs between conditions: pneumonia vs. healthy control. Downregulated genes are colored blue and upregulated genes are colored red (thresholds: adjusted *P*-value = 0.05, log₂FC = 121). **(C)** Correlation between log₂FC of DEGs obtained from the comparison pneumonia vs healthy controls in RNA-seq/Microarray data. Only genes with adjusted *P*-value < 0.05 and log₂FC > |1| are displayed. The color scale represents the differences between the log₂FC values of both analyses. The *P*-value of the correlation is 2.2e⁻¹⁶ and only names of genes with a Log₂FC > 151 are shown. **(D)** Two-way hierarchical clustering analysis heat map of DEGs between pneumonia and healthy control samples in RNA-seq and microarray validation cohorts. Each row represents one transcript; each column represents one patient. The bar at the bottom indicates the sample phenotype. Only genes with a log₂FC > |1| and adjusted *P-value <* 0.05 were represented in the heatmap and only the genes that where common in the top 40 with the lower *P-value* in both analysis where printed. Expression intensity is indicated by color (Red = high expression, Blue = low expression). **(E)** Dot plot from ORA pathway analysis of common DEGs between RNA-seq and microarray cohort with adjusted *P*-value (FDR) < 0.05 and a log₂FC > 11.51 for the comparison pneumonia patients vs. Healthy controls using Reactome as reference. Size along the x-axis indicates the number of genes in the input list that are annotated to the corresponding term / number of genes in input list (gene ratio). Dot colors correspond to the different FDR *P*-values associated with the pathways. **(F)** Dot plot from ORA pathway analysis of common DEGs between RNA-seq and microarray cohort with FDR *P-*value < 0.05 and a log₂FC > 11.51 for the comparison pneumonia patients *vs*. Healthy controls using GO as reference. Size along the x-axis indicates the number of genes in the input list that are annotated to the corresponding term / number of genes in input list (gene ratio). Dot colors correspond to the different FDR *P*-values associated with the pathways.

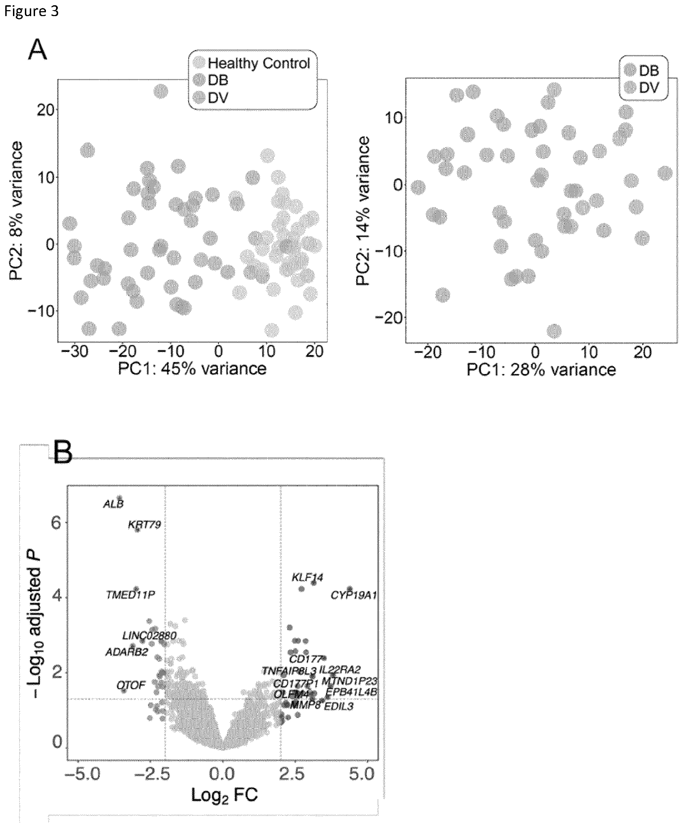

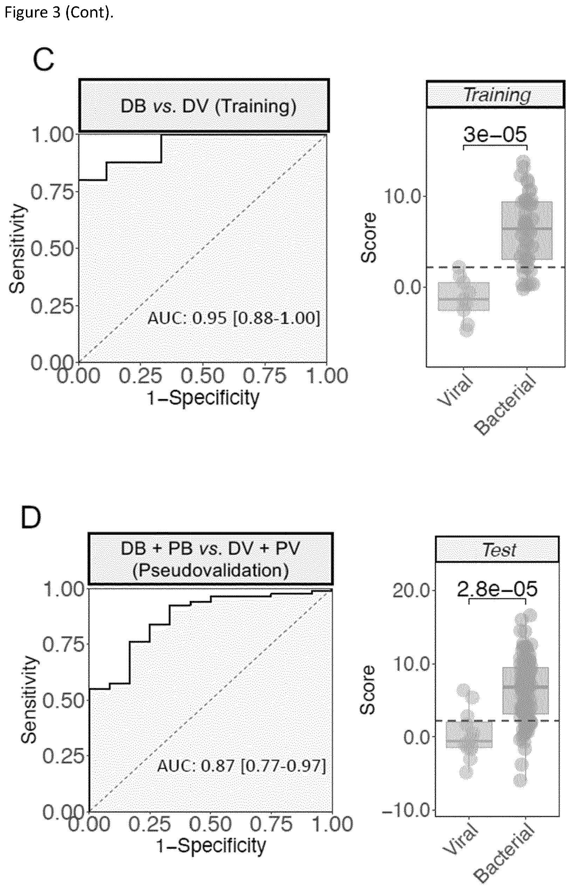

**Figure 3**.**(A)**PCA of transcriptome profiles of DV (definitive viral) and DB (definitive bacterial) pneumonias and healthy control samples. Two first principal components (PC1 and PC2) are shown. **(B)** Volcano plot showing the DEGs between conditions: DB pneumonia vs. DV pneumonia (DV). Downregulated genes are colored blue and upregulated genes are colored red (thresholds: adjusted *P*-value = 0.05, log₂FC = |2|). **(C)** Receiver operating characteristic curves (ROC) based on the specific 5-transcript signature from the training cohort including the area under the curve (AUC) and 95% confident intervals (CIs) values (left). Boxplots of the predicted values using the optimal model in training cohort. Wilcoxon *P*-value is also displayed (right). **(D)**Receiver operating characteristic curves (ROC) based on the specific 5-transcript signature from test cohort including the area under the curve (AUC) and 95% confident intervals (CIs) values (left). Boxplots of the predicted values using the optimal model in training cohort. Wilcoxon *P*-value is also displayed (right). Red dashed line represents the optimal cutpoint.

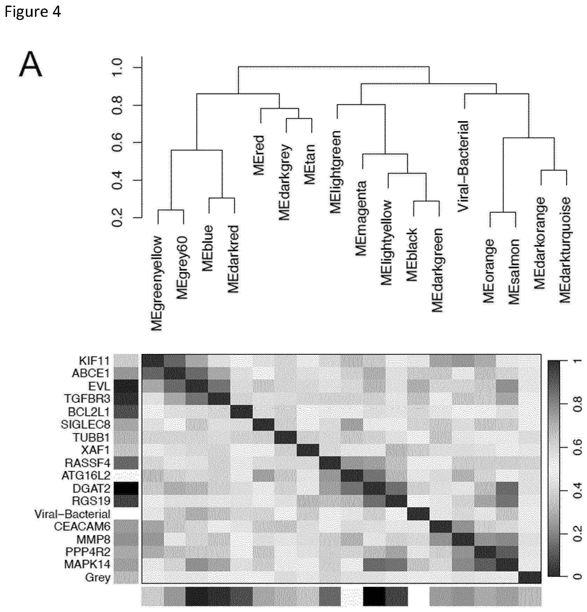

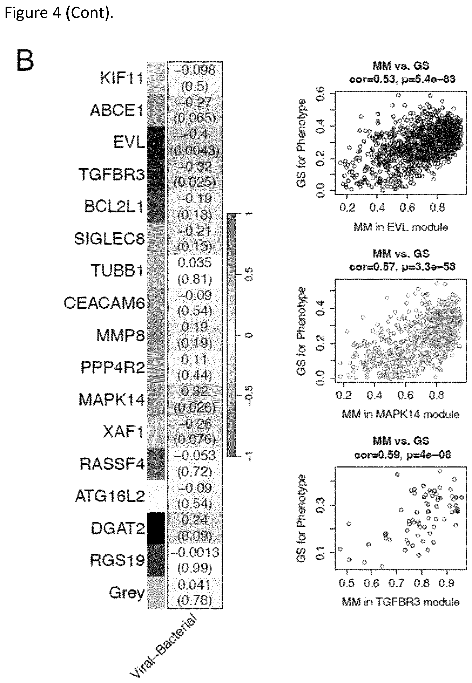

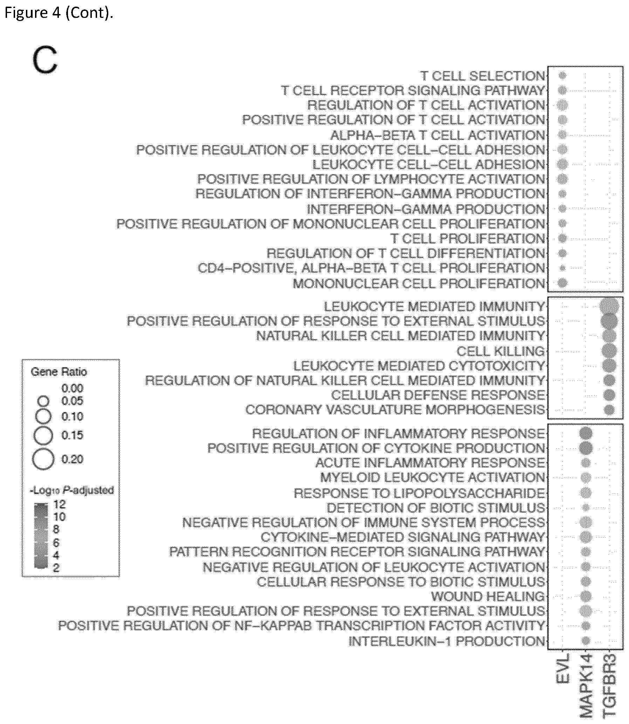

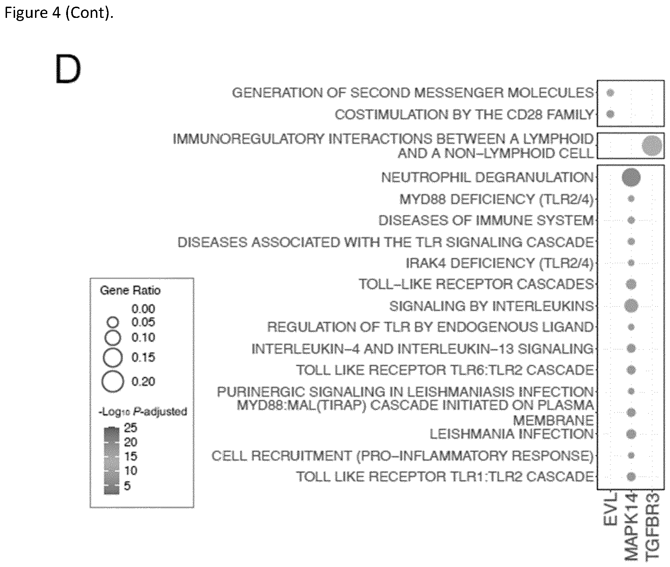

**Figure 4**.**(A)** Hierarchical clustering eigengene dendrogram and heatmap showing relationships among the modules and pneumonia etiology (DB and DV pneumonias). Gene names on the left of the heatmap are the hub genes of each module. **(B)** Correlation values heatmap between co-expression modules and DB and DV conditions. Upper value shows to individual correlation value of the module with musical stimuli (left). *P*-values of these correlations are represented in brackets (lower values). Plots showing comparison between MM (module membership) and GS (correlation with bacterial/viral condition) of genes from the most significant modules detected. **(C)** Top 15 statistically associated GO biological processes detected for each of the significantly correlated modules **(D)** Top 15 statistically associated Reactome pathways detected for each of the significantly correlated modules.

### Detailed description of the invention

The present invention is illustrated by means of the Examples set below without the intention of limiting its scope of protection.

### Example 1. MATERIAL AND METHODS

### Example 1.1. Study design

This study utilized blood samples from paediatric patients recruited in the European Union Childhood Life-threatening Infectious Diseases Study (EUCLIDS- https://www.euclids-project.eu/). The EUCLIDS study enrolled children and young adolescents aged 1 month to 18 years who were admitted to hospitals with suspected sepsis or severe focal infection. The study encompassed a network of 194 hospitals across nine European countries, as well as two hospitals located in The Gambia, Africa. Patient recruitment occurred between July 1, 2012, and December 31, 2015.

Pneumonia phenotype was defined as clinical symptoms compatible with acute respiratory infection and inflammation of one or both lungs with lobar or segmental or multilobar collapse/consolidation on CXR. More specifically, following radiological findings of consolidation/pleural effusion must be met: alveolar consolidation (defined as a dense or fluffy opacity that occupies a portion or whole of a lobe or of the entire lung that may or may not contain air-bronchograms) or pleural effusion (defined as fluid in the lateral pleural space and not just in the minor or oblique fissure) that was spatially associated with a pulmonary parenchymal infiltrate (including other infiltrate) or obliterated enough of the hemithorax to obscure an opacity. It does not include just perihilar consolidation or patchy consolidation. Children with pneumonia *(n =* 154) and age and sex matched healthy controls *(n* = 38; **Table 2**) were selected from the EUCLIDS database for transcriptome analysis in whole blood samples collected in PAXgene^{™} and Tempus^{™} RNA tubes. **Table 2** shows clinical features of the pneumonia patient's cohort. Wilcoxon and Fisher exact test were used to assess statistical significance between groups in numerical and categorical variables respectively. IV: invasive ventilation; NIV: non-invasive ventilation (*) indicates statistical significance.

**Table 2**

| | **Pneumonia *vs.* Healthy controls** | | | **DV *vs*. DB** | | | | | **DV** + **PV *vs*. DB** + **PB** | | | | |
|---|---|---|---|---|---|---|---|---|---|---|---|---|---|
| | **Controls (*n* = 38)** | **Pneumonia (*n* = 154)** | ***n*** | **DB (*n* = 40)** | ***n*** | **DV (*n*= 9)** | ***n*** | ***P-*value** | **DB + PB (*n* = 98)** | ***n*** | **DV + PV (*n =* 12)** | ***n*** | ***P-*value** |
| **Gender (Male)** | 68.4% (36/38) | 51.3% (79/154) | 154 | 62.5% (25/40) | 40 | 55.6% (5/9) | 9 | 0.72 | 53.1% (52/98) | 98 | 58.3% (7/12) | 12 | 0.769 |
| **Age (years)** | 3.3 (2.3-8.2) | 3.36 (1.24-6.17) | 153 | 2.77 (1.25-5.78) | 40 | 2.75 (1.52-4.76) | 8 | 0.923 | 3.78 (1.54-6.285) | 98 | 1.93 (0.94-3.88) | 11 | 0.096 |
| **Days hospitalized** | - | 5 (3-10) | 149 | 6.5 (3.5-14) | 38 | 6 (4.75-8.5) | 8 | 0.621 | 5 (3-9) | 95 | 6 (3.5-9) | 11 | 0.536 |
| **PICU admission** | - | 24.7% (38/154) | 154 | 30% (12/40) | 40 | 22.2% (2/9) | 9 | 1 | 22.4% (22/98) | 98 | 25% (3/12) | 12 | 1 |
| *PICU days* | - | 7 (3-11) | 37 | 11 (6.5-16) | 11 | 6.5 (4.25-8.75) | 2 | 0.372 | 6 (3-11) | 21 | 2 (2-6.5) | 3 | 0.356 |
| **Death** | - | 3.2% (5/154) | 154 | 7.5% (3/40) | 40 | 11.1% (1/9) | 9 | 0.569 | 4.1% (4/98) | 98 | 8.3% (1/12) | 12 | 0.445 |

| **Treatments** | | | | | | | | | | | | | |
|---|---|---|---|---|---|---|---|---|---|---|---|---|---|
| *Inotropes* | - | 9.7% (15/154) | 154 | 5% (2/40) | 40 | 11.1% (1/9) | 9 | 0.464 | 6.1% (6/98) | 98 | 8.3% (1/12) | 12 | 0.565 |
| *IV* | - | 16.2% (25/154) | 154 | 15% (6/40) | 40 | 22.2% (2/9) | 9 | 0.628 | 11.2% (11/98) | 98 | 25% (3/12) | 12 | 0.18 |
| *NIV* | - | 11% (17/154) | 154 | 15% (6/40) | 40 | 11.1%(1/9) | 9 | 1 | 8.2% (8/98) | 98 | 8.3% (1/12) | 12 | 1 |
| *Oxygen* | - | 39% (60/154) | 154 | 40% (16/40) | 40 | 33.3% (3/9) | 9 | 1 | 34.7% (34/98) | 98 | 41.7% (5/12) | 12 | 0.751 |

| **Analytical parameters** | | | | | | | | | | | | | |
|---|---|---|---|---|---|---|---|---|---|---|---|---|---|
| *CRP (mg*/*L)* | - | 81 (30.1-215.5) | 59 | 146 (34.5-244) | 11 | 28.5 (11.85-30.15) | 6 | 0.062 | 190 (81-273) | 33 | 27 (5.9-30.1) | 7 | 0.001* |
| *Platelets (10⁹*/*L)* | - | 322 (259-433) | 135 | 287.5 (175.5-405.5) | 28 | 491 (283-507) | 5 | 0.173 | 324 (262.75-421.5) | 84 | 398 (273.5-495) | 8 | 0.458 |
| *White Cells* /*10⁹*/*L)* | - | 17.2 (10.2-25) | 133 | 18.75 (10.075-26.075) | 28 | 7.2(6.35-16.25) | 3 | 0.423 | 18.65 (12.18-26.72) | 84 | 6.85 (5.75-20.775) | 6 | 0.089 |
| *Lactate (mmol*/*L)* | - | 1.9 (1.18-2.65) | 30 | 2.5 (2.3-2.7) | 5 | 2.5 (2.5-2.5) | 1 | 1 | 2.2 (1.48-2.55) | 16 | 2.5 (2.5-2.5) | 1 | 0.539 |
| *Neutrophils (10⁹*/*L)* | - | 11.87 (6.47-19.95) | 128 | 13.4 (8.05-20.88) | 26 | 1206 (7.94-16.18) | 2 | 0.762 | 14.61 (7.82-23.62) | 82 | 4.16 (3.82-18.6) | 5 | 0.111 |
| *Monocytes (10⁹*/*L)* | - | 0.8 (0.17-1.7) | 84 | 0.61 (0.125-1.2) | 14 | 0.8 (0.4-1.15) | 3 | 0.801 | 0.955 (0.168-1.92) | 56 | 0.02 (0-0.8) | 5 | 0.068 |
| *Lymphocytes (10⁹*/*L)* | - | 3.17 (1.67-5.4) | 130 | 1.95 (1.4-5.65) | 27 | 4.1 (3.42-8.55) | 3 | 0.213 | 2.6 (1.5-5.4) | 83 | 3.42 (2.1475-6.35) | 6 | 0.381 |

In a subsequent analysis, pneumonia samples were further classified using the algorithm developed by Herberg et al. C-reactive protein (CRP) and neutrophil threshold criteria were employed where available, with 72.5% and 33.3% of missing CRP values in DB and DV, respectively. Definitive Bacterial (DB; *n* = 40) and Definitive Viral (DV; *n* = 9) pneumonias were selected to study the differences in transcriptome depending on the etiology of the infection. Pneumonias categorized as Probable Bacterial (PB) and Probable Viral (PV) were excluded from this specific analysis to minimize possible confounding factors in stablishing a transcriptomic signature for differentiating bacterial from viral pneumonia in children but were used post-hoc to validate the signature in a more realistic clinical scenario (**Figure 1**).

### Example 1.2. RNA-seq analysis

RNA was isolated from blood samples collected in PAXgene^{™} and in Tempus^{™} RNA tubes. RNA sequencing was conducted on a HiSeq 4000 (Illumina) platform, with library preparation and sequencing of 30 million 75 or 100 bp paired end reads. The Illumina's TruSeq RNA Sample Preparation Kit was used for library preparation, and ribosomal and globin RNA depletion was performed using the Illumina^{®} Ribo-Zero Gold kit. Quality control of raw data was carried out using *FastQC,* alignment and read counting were performed using *STAR,* alignment filtering was done with *SAMtools* and read counting was carried out using *FeatureCounts.*

### Example 1.3. Differential Expression Analysis

Data normalization, differential expression (DE) analysis and covariates correction were carried out using *DESeq2* and *RUVSeq* R packages. Differentially expressed genes (DEGs) were identified through the Negative Binomial distribution implemented in the *DESeq* package. The fitted model included known covariates such as sex, batch, and type of collection tube, to correct the differences in gene expression related to these factors. The *RUVg* method implemented in the *RUVSeq* package of R was employed to identified and adjust for other factors of undesirable variation present in the dataset, selecting a *k* = 2 as a number of variables to include. A generalized linear model was fitted, and a *t*-statistic was calculated for each gene. *P*-values were corrected for multiple testing using the Benjamini-Hochberg False Discovery Rate (FDR).

As a validation cohort for the pneumonia vs. control study, we downloaded the GSE103119 microarray dataset from the Gene Expression Omnibus (GEO) database. This dataset comprises whole blood gene expression data from children with pneumonia (*n* = 152) as well as healthy controls *(n =* 20). Data were normalized, processed and analyzed using the *limma* R package. A linear model was fitted, considering the sex as a categorical covariate. Multiple testing correction was performed using the Benjamini-Hochberg False Discovery Rate (FDR). Principal Component Analysis (PCA) was used to visualize the global transcriptome patterns of both RNA-seq and microarray data. A Spearman test was computed for the correlation indices (ρ) and the associated *P*-values. Wilcoxon test was performed to assess statistical significance between groups.

### Example 1.4. Pathway Analysis

We used the Reactome and GO (Gene Ontology) databases as references to examine biological pathways and processes associated with the DEGs in pneumonia patients. We followed an over-representation analysis (ORA) approach, considering DEGs with a log₂FC ≥ |1.5| and a adjusted *P*-value < 0.05. The analysis was performed using the *Clusterprofiler* R package. To account for multiple test correction, we applied the Benjamini-Hochberg (H-B) procedure, establishing a significant threshold of 0.05.

### Example 1.5. Signature Discovery using LASSO Regression Model

We employed a LASSO regression model to identify a subset of genes that could serve as a predictive transcriptomic signature differentiating viral from bacterial pneumonia in the RNA-seq cohort. A predictive transcriptomic signature was computed using the R package *glmnet.* A logistic LASSO regression model was fitted with the alpha parameter set to 1. We selected 36 DEGs filtered based on |log₂FC| > 1.5, adjusted *P*-value < 0.05, and a Base Mean > 50 as input for the model. To determine the optimal parameter selection for the LASSO regression model, we conducted a 10-fold cross-validation, which helps evaluate the model's performance and effectively tune the parameters.

To assess the accuracy of the predictive transcriptomic signature, we calculated the area under the receiver operating characteristic curve (AUC) with 95% confidence intervals (CI) using the *pROC* package in R. Receiver Operating Characteristic (ROC) curves were generated to graphically represent the model's true positive rate against the false positive rate. The optimal cut-point value, maximizing sensitivity and specificity, was calculated using the Youden method included in the *OptimalCutPoints* R package.

### Example 1.6. Weighted Gene Correlation Network Analysis (WGCNA)

We conducted a co-expression network analysis using the *WGCNA* package to detect clusters of co-expressed genes specifically correlated to viral or bacterial pneumonia. We selected all patients with DV *(n =* 9) and DB *(n =* 40) pneumonia for the analysis. Only protein-coding genes exhibiting the highest expression variance among samples (the top 25%) were included, totaling 4,831 genes. Normalized gene expression data were used to construct a signed weighted correlation network. A matrix of correlations between all pairs of selected genes was generated from the expression values and converted into an adjacency matrix with a power function. We determined a soft-thresholding power of 20 (maximum model fitting index), selected on the criterion of scale-free topology after evaluating several candidate powers. We calculated the Topological Overlap Matrix (TOM) and the corresponding dissimilarity (1-TOM) values. For module detection and merging, we showed a minimum module size of 30, and a cut height threshold of 20, respectively. Correlation between module eigengenes and the viral/bacterial pneumonia was calculated to identify modules of co-expressed genes significantly associated with the phenotype (gene significance, or GS). Module Membership (MM) measured intramodular connectivity, and the most interconnected gene (hub gene) named the modules.

The biological pathways represented by each of the significantly correlated modules were investigated through an over-representation analysis with the *Clusterprofiler* R package, with terms from GO and Reactome databases as references.

All graphics were created using R software v.4.2.0 (www-r-project.org).

### Example 2. RESULTS

### Example 2.1. Cohort description

In this study, blood samples were collected from 192 children. Among them, 154 were hospitalized with pneumonia (median age: 3.4 years; 51.3% male) and 38 were healthy controls (median age: 3.3 years; 68.4% male). Clinical and demographic details of the entire cohort and sub-categories used in different analyses are provided in **Table 2.** Among the pneumonia patients, 25% were admitted to PICU, 3% died, and 39% required oxygen support (16.2% with invasive ventilation). No statistically significant differences were found between the bacterial and viral pneumonia sub-groups for these parameters. The majority of bacterial pneumonias were caused by *Streptococcus pneumoniae* (47.5%) followed by *Staphylococcus aureus* (10.0%*) and Streptococcus pyogenes* (7.5%) *between others* (35.0%). Viral pneumonias were caused by influenza virus (22.2%), bocavirus (22.2%), respiratory syncytial virus (22.2%), rhinovirus (11.1%), adenovirus (11.1%) and parainfluenza (11.1%).

As expected, C-reactive protein (CRP) values were significantly higher in DB + PB compared to DV + PV (*P*-value = 0.001). CRP values were higher in the DB group compared to DV; this difference approached the significance threshold (*P*-value = 0.062), likely due to limited statistical power, especially given the low number of samples in DV group, coupled with missing data. For the remaining blood tests, there were no significant statistical differences between bacterial and viral sub-groups.

### Example 2.2. Differentially expressed genes in pneumonia

Through a comparative analysis of transcriptomes in children with pneumonia vs. healthy controls, we identified 5,486 DEGs, using a significance threshold of FDR 5%. Within this set of DEGs, 2,716 were found to be upregulated, while 2,770 were downregulated.

To gain insight into the overall variation in gene expression, we performed a PCA on a subset of 100 highly variable genes out of a total of 192 samples. The first principal component (PC1), explaining approximately 30% of the variation, effectively segregated the samples into two main clusters, corresponding to pneumonia samples and healthy control samples (**Figure 2A**).

To prioritize significant changes in gene expression, we applied a selective criterion, including an adjusted *P*-value < 0.05 and a log₂FC > 121. This selection process yielded 272 DEGs (**Figure 2B****),** with 208 exhibiting upregulation and 64 genes showing downregulation, indicating a prevailing pattern of overexpression among these genes.

To validate the DEG findings obtained from our RNAseq cohort, we compared them with data from a study conducted by Wallihan et al. (accession number GSE103119). The dataset by Wallihan et al. included transcriptomic data from blood samples of pediatric pneumonia cases (*n* =152) and healthy controls (*n* = 20). This comparison revealed 1,729 common DEGs. Notably, the comparison of DEGs exhibiting significant expression changes in relation to healthy controls (adjusted *P*-value < 0.05 and log₂FC > |1|; *n* = 310 genes) from our study and Wallihan's et al. data demonstrated a high positive correlation (ρ = 0.81; *P*-value < 2.2e⁻¹⁶) between log₂FC values obtained (**Figure 2C**). These 310 common genes' expression patterns segregated the samples into two distinct clusters, aligning with pneumonia and healthy control groups in both our RNA-seq discovery cohort and the validation microarray dataset (**Figure 2D**).

### Example 2.3. Functional enrichment analysis of differentially expressed genes

To further characterize DEGs from a functional point of view, we conducted an over-representation analysis (ORA) based on GO and Reactome using only validated DEGs with a positive correlation between the discovery and the validation datasets. We set a threshold of an adjusted *P*-value < 0.05 for significance and a |log₂FC| > 1.5. The analysis revealed that the most significant pathways (adjusted *P*-value < 0.01) are associated with essential immune processes, primarily related to the innate response to severe CAP (**Figure 2E, Figure 2F**). The Reactome-ORA analysis indicated a robust association between the selected DEGs, including *MMP8, OLFM4* or *CD177,* and an innate response characterized by 'neutrophil degranulation'. This pathway displayed the highest significance (adjusted *P*-value = 6.5e⁻¹²). Consistently, GO-ORA results identified differences in neutrophil-related immune pathways, including `leukocyte mediated immunity' (adjusted *P*-value = 7.4e⁻⁵), `myeloid leukocyte mediated immunity' (adjusted *P*-value = 0.001), 'neutrophil mediated immunity' (adjusted *P-*value = 5.1e⁻⁵), `myeloid leukocyte activation' (adjusted *P*-value = 1.4e⁻⁵), `granulocyte activation' (adjusted *P*-value = 0.002), and `neutrophil activation' (adjusted *P*-value = 0.001) (**Figure 2E, Figure 2F**).

Furthermore, there is a significant overlap in enrichment results from GO and Reactome, particularly regarding humoral immunity processes. Notably, GO-ORA identified 'humoral immune response' (adjusted *P*-value = 4.4e⁻⁵), `antimicrobial humoral response' (adjusted *P-*value = 1.4e⁻⁵) and `antimicrobial humoral immune response mediated by antimicrobial peptide' (adjusted *P*-value = 0.0003) (**Figure 2E, Figure 2F**) as highly significant pathways. Likewise, Reactome-ORA highlighted the involvement of various humoral responses in severe CAP, including the complement system (`regulation of complement cascade' [adjusted *P*-value = 0.001] and `complement cascade' [adjusted *P*-value = 0.003]), as well as humoral immunity mediated by `antimicrobial peptides' (adjusted *P*-value = 1.2e⁻⁹) (**Figure 2E, Figure 2F**). Most significant pathways from GO-ORA were those implicated in `defense response to bacterium' (adjusted *P*-value = 2.3e⁻⁸), 'defense response to fungus' (adjusted *P*-value = 1.0e⁻⁵) and related processes ('defense response to Gram-negative bacterium' and `antibacterial humoral response') (**Figure 2E, Figure 2F**). It is not surprising that genes related to defense against bacteria were overrepresented in the overall analysis as most of the samples in the dataset correspond to bacterial origin pneumonia.

Additionally, GO-ORA analysis highlighted a significant association of CAP with biological processes that play essential roles not only in innate protection against viral and bacterial invasion but also in disease pathogenesis. This encompasses regulatory pathways related to transcription factors, such as the pro-inflammatory NF-kappaB ('regulation of DNA-binding transcription factor activity', `positive regulation of DNA-binding transcription factor activity', `positive regulation of NF-kappaB transcription factor activity') as well as pathways indicating inflammatory response ('regulation of inflammatory response', `positive regulation of inflammatory response' and `acute inflammatory response') and activation of the cytokine signalling system ('positive regulation of cytokine production' and `cytokine-mediated signaling pathway') (**Figure 2E, Figure 2F**).

### Example 2.4. Bacterial vs. viral pneumonia: DEGs and diagnostic signature

We conducted an additional PCA analysis to compare the transcriptomic profiles of pneumonia in DB and DV infections. Our goal was to investigate how these two groups cluster in relation to each other and, in comparison, to the control group. In the first PCA (**Figure 3A**), pneumonias separated distinctly from the control group along PC1, which accounted for 45% of the variation. Specifically, the DB clustered at one pole of the plot, the controls in the opposite side, and the DV profiles in between. A separate PCA including only DB (*n* = 40) and DV (*n* = 9) transcriptomic profiles samples, revealed a relatively weak separation between the two groups. DV profiles primarily clustered to one side along its PC1 (28% of the variation), albeit with some slight mixing with other DB samples; **Figure 3A****.**

Our differential expression analysis between DB and DV pneumonias identified 282 DEGs (FDR of 5%). The majority showed under-expression in DB with respect to DV pneumonia (*n* = 217; 77%). However, when considering genes with more substantial expression changes (setting a threshold of adjusted *P*-value < 0.05 and a |log₂FC| > 2), we found that the proportion of over-expressed genes in DB pneumonia (*n* = 30; 55%) was slightly higher than the proportion of under-expressed genes (*n* = 25; 45%); **Figure 3B****.** Interestingly, *ALB* was the gene yielding the lowest adjusted *P*-value (1.88e-7) in the comparison between DB and DV patients (**Figure 3B**).

To further investigate, we selected a sub-set of DEGs candidates from the DV vs. DB differential expression in pneumonia samples (*n* = 36; see Material and Methods). We then employed the lasso regression algorithm to identify the optimal transcriptomic signature for distinguishing between these two types of infection. Due to the limited number of samples classified as viral pneumonia (DV = 9; PV = 3), we were unable to split the dataset into training and test sets conventionally. Instead, we adopted a pseudo-validation approach. This approach included only samples confidentially classified as DB and DV in the training dataset (DB = 40; DV = 9), and all DB and PB and viral pneumonia samples in test dataset to validate the performance of the transcriptomic signature (DB + PB = 98; DV + PV = 12).

The best model for differentiation between viral and bacterial pneumonia comprised five genes *(FAM20A, BAG3, TDRD9, MXRA7* and *KLF14*), forming the optimal signature (**Table 3**). **Table 3** shows the regression coefficients for the 5-transcript signature for differentiating viral from bacterial pneumonia. Among these genes, four were over-expressed (*FAM20A, TDRD9, MXRA7* and *KLF14*) while one (*BAG3*) showed under-expression in bacterial compared to viral pneumonia. This transcriptomic signature statistically discriminated between viral and bacterial pneumonia with high accuracy (*P*-value = 3.0e⁻⁵; optimal cut-off value = 2.204; **Figure 3C****)** and demonstrated excellent performance in the training set, generating an AUC value of 0.95 [0.88-1.00] (sensitivity = 0.80 and specificity = 1.00) (**Figure 3C**).

**Table 3**

| **Gene symbol** | ***FAM20A*** | ***BAG3*** | ***MXRA7*** | ***TDRD9*** | ***KLF14*** | **(Intercept)** |
|---|---|---|---|---|---|---|
| **Coefficient** | 0.05964 | -1.25018 | 2.18318 | 1.57463 | -0.53523 | -16.31345 |

This 5-transcripts signature identified in the training cohort exhibited robust discriminatory capacity when validated in the test set (*P*-value = 2.8e⁻⁵, **Figure 3D**), which included PV and PB pneumonia samples. In this scenario, the ROC curve also indicates high accuracy, resulting in an AUC value of 0.87 [0.77-0.97] (sensitivity = 0.76 and specificity = 0.83); **Figure 3D****.**

Additionally, we also tested our transcriptomic signature in a new dataset containing only PV and PB samples and we obtained an expected decrease in the AUC value (0.69 [0.40-0.97]), as these samples are coded with a phenotype of lower clinical confidence, and are therefore more likely to contain viral samples within the PB set, bacterial samples within the PV set, or mixed infections (see score values for classification in **Figure 3C** and **3D**).

Furthermore, we compared the performance of our 5-transcriptomic signature with the previously described 2-transcript signature that generically differentiate viral from bacterial infections in febrile children using *IFI44L* and *FAM89A* transcripts. Although the 2-transcript signature showed a good performance discriminating viral and bacterial pneumonia (AUC: 0.87 [0.76-0.96]) in our discovery dataset, the AUC value was lower than that obtained from the specific 5-transcript signature we propose. When we tested the 2-transcripts viral/bacterial signature in the PB vs. PV subset, the AUC value was also lower than the AUC from the pneumonia signature (AUC:0.44 [0.00-0.89]). Finally, to check the best performance that we could achieve with these two transcripts in our discovery dataset, we re-trained the model using *IFI44L* and *FAM89A* transcripts. In this case we obtained an AUC value of 0.88 [0.77-0.98], which is still below the AUC from the specific pneumonia signature (AUC: 0.95 [0.88-1.00]). Finally, we have also tested the 5-transcripts signature and the 2-transcript signature from Herberg et al. [Herberg JA, Kaforou M, Wright VJ, Shailes H, Eleftherohorinou H, Hoggart CJ, et al. Diagnostic test accuracy of a 2-transcript host RNA signature for discriminating bacterial vs viral infection in febrile children. Jama. 2016;316(8):835-45] in the bacterial and viral pneumonia samples (excluding co-infections) from dataset of Wallihan et al. [Wallihan RG, Suárez NM, Cohen DM, Marcon M, Moore-Clingenpeel M, Mejias A, et al. Molecular distance to health transcriptional score and disease severity in children hospitalized with community-acquired pneumonia. Frontiers in cellular and infection microbiology. 2018; 8: 382*].* Surprisingly, the two signatures did not work at all, yielding AUC values close to 0.5 in both cases, in this dataset.

### Example 2.5. Co-expression analysis of viral vs. bacterial pneumonia

To understand the distinct host mechanisms and key genes involved in the specific response to viral and bacterial pneumonia, we conducted a co-expression network analysis using the sub-set of pneumonia samples with DB or DV origin. The analysis detected 16 modules of co-expressed genes, with three of them significantly correlated (*P*-value < 0.05) with viral/bacterial phenotypes (**Figure 4A****;** **Figure 4B**). The blue module ([EVL module], *R* = - 0.4; *P*-value = 0.0043; 661 genes) and darkred module ([TGFBR3 module], *R* = -0.32; *P*-value = 0.025; 73 genes) exhibited negative correlation with bacterial pneumonia. In contrast, the salmon module [MAPK14] showed a positive correlation (*R* = 0.32; *P*-value = 0.026; 1,132 genes) with bacterial pneumonia (**Figure 4A****;** **Figure 4B**)**.** The EVL and TGFBR3 modules clustered together on the dendrogram, indicating their involvement in similar biological processes globally activated during viral pneumonia **(****Figure 4A****).** Genes within these significant modules displayed a strong correlation between trait significance (GS) and module membership (MM), suggesting that highly interconnected genes (higher MM) within the module are closely related to the causal pathogen of pneumonia (**Figure 4B**). These driver genes, located in the upper right side of the plots, are pivotal for predicting viral or bacterial pneumonia.

Functional assessment of the significant modules unveiled different immune responses for viral and bacterial pneumonia. The modules over-regulated in viral pneumonia showed enrichment in terms associated with both innate and adaptative immune responses. For example, the EVL module was linked to adaptative T-cell response (T-cell selection, T-cell receptor signalling, T-cell activation, differentiation, and proliferation) and interferon production (interferon gamma production) (**Figure 4C**), while the TGBR3 module participated in `natural killer mediated immunity' (innate response) and, as in the case of the EVL module, in an adaptative response involving `leukocyte mediated cytotoxicity' and `T-cell mediated immunity'. Conversely, the significant pathways related to genes from the bacterial MAPK14 module primarily represent a different innate response characterized by `positive regulation of cytokine production', `cytokine-mediated signalling pathway', `acute inflammatory response', `pattern recognition receptor signalling' and `myeloid leukocytes activation' (Granulocytes, monocytes, macrophages, and dendritic cells). These findings were further supported by an over-representation analysis using the Reactome database, emphasizing the role of MAPK14 module genes in innate immune responses, including neutrophil degranulation, various toll-like receptor (TLR) cascades, and signalling by interleukins (**Figure 4D**).

## Claims

1. *In vitro* method for identifying biomarkers or biomarker signatures for the differential diagnosis between viral and bacterial pneumonia in a patient, which comprises assessing the level of expression of at least a gene selected from the group consisting of: *MXRA7*, *FAM20A, BAG3, TDRD9* or *KLF14,* in a biological sample obtained from the patient, wherein the identification of a deviation of the level of expression of the gene/s as compared with a pre-established level of expression measured in a biological sample obtained from patients with viral pneumonia, is an indication that the gene/s may be used as biomarker or biomarker signatures for the differential diagnosis between viral and bacterial pneumonia.

2. *In vitro* method for the differential diagnosis between viral and bacterial pneumonia in a patient, which comprises assessing the level of expression of at least a gene selected from the group consisting of: *MXRA7, FAM20A, BAG3, TDRD9* or *KLF14* in a biological sample obtained from the patient, wherein the identification of an over-expression of the genes *MXRA7*, *FAM20A, TDRD9* or *KLF14,* or an under-expression the gene *BAG3,* as compared with a pre-established level of expression measured in a biological sample obtained from patients with viral pneumonia, is an indication that the patient is suffering from bacterial pneumonia; or wherein the identification of an under-expression of the genes *MXRA7*, *FAM20A, TDRD9* or *KLF14,* or an over-expression the gene *BAG3,* as compared with a pre-established level of expression measured in a biological sample obtained from patients with bacterial pneumonia, is an indication that the patient is suffering from viral pneumonia.

3. *In vitro* method for selecting a therapy for a patient suffering from pneumonia which comprises assessing the level of expression of at least a gene selected from the group consisting of: *MXRA7*, *FAM20A, BAG3, TDRD9* or *KLF14* in a biological sample obtained from the patient, wherein the identification of an over-expression of the genes *MXRA7*, *FAM20A, TDRD9* or *KLF14,* or an under-expression the gene *BAG3,* as compared with a pre-established level of expression measured in a biological sample obtained from patients with viral pneumonia, is an indication that the patient if suffering from bacterial pneumonia and consequently a treatment with antibiotics can be recommended; or wherein the identification of an under-expression of the genes *MXRA7*, *FAM20A, TDRD9* or *KLF14,* or an over-expression the gene *BAG3,* as compared with a pre-established level of expression measured in a biological sample obtained from patients with bacterial pneumonia, is an indication that the patient is suffering from viral pneumonia and consequently a treatment with antibiotics can be discarded.

4. *In vitro* use of at least a gene selected from the group consisting of: *MXRA7, FAM20A, BAG3, TDRD9* or *KLF14,* or of a kit comprising reagent for assessing the level of expression of at least a gene selected from the group consisting of: *MXRA7, FAM20A, BAG3, TDRD9* or *KLF14,* for identifying biomarkers or biomarker signatures for the differential diagnosis between viral and bacterial pneumonia in a patient, for the differential diagnosis between viral and bacterial pneumonia in a patient or for selecting a therapy for a patient suffering from pneumonia.

5. Antibiotic for use in a method for the treatment of pneumonia wherein the method comprises determining whether the patient is suffering from bacterial pneumonia by assessing the level of expression of at least a gene selected from the group consisting of: *MXRA7*, *FAM20A, BAG3, TDRD9* or *KLF14* in a biological sample obtained from the patient, wherein the identification of an over-expression of the genes *MXRA7, FAM20A, TDRD9* or *KLF14,* or an under-expression the gene *BAG3,* as compared with a pre-established level of expression measured in a biological sample obtained from patients with viral pneumonia, is an indication that the patient is suffering from bacterial pneumonia and consequently a treatment with antibiotics can be recommended.

6. *In vitro* method according to claims 1 to 3, *in vitro* use according to claim 4, or antibiotics for use according to claim 5, wherein the biomarker or biomarker signature is selected from **Table 1.**

7. *In vitro* method according to claims 1 to 3, *in vitro* use according to claim 4, or antibiotics for use according to claim 5, which comprises assessing the level of expression of the combination of genes: *MXRA7*, *FAM20A, BAG3, TDRD9* and *KLFl4* in a biological sample obtained from the patient, wherein the identification of an over-expression of the genes *MXRA7, FAM20A, TDRD9* and *KLF14,* and an under-expression the gene *BAG3,* as compared with a pre-established level of expression measured in a biological sample obtained from patients with viral pneumonia, is an indication that the patient if suffering from bacterial pneumonia; or wherein the identification of an under-expression of the genes MXR,4 7, *FAM20A, TDRD9* and *KLF14,* and an over-expression the gene *BAG3,* as compared with a pre-established level of expression measured in a biological sample obtained from patients with bacterial pneumonia, is an indication that the patient is suffering from viral pneumonia.

8. *In vitro* method according to claims 1 to 3, *in vitro* use according to claim 4, or antibiotics for use according to claim 5, wherein the biological sample is selected from blood, serum or plasma.

9. Antibiotics for use, according to claim 5, selected from the group: amoxicillin, azithromycin, ampicillin or cefotaxime.
